# EUROPÄISCHE PATENTSCHRIFT

(11) **EP 1 067 965 B1**
(45) Veröffentlichungstag und Bekanntmachung des Hinweises auf die Patenterteilung: **29.09.2004**
(21) Anmeldenummer: 98958922.1
(22) Anmeldetag: 26.11.1998
(51) Int. Cl.: A61K 45/06, A61P 25/00

(54) **PHARMAZEUTISCHE ZUSAMMENSETZUNG ZUR BEHANDLUNG VON SCHLAGANFALL UND SCHÄDEL-HIRN-TRAUMA**
PHARMACEUTICAL COMPOSITION FOR TREATING CEREBRAL APOPLEXY AND CRANIOCEREBRAL TRAUMA
COMPOSITION PHARMACEUTIQUE POUR TRAITER L'APOPLEXIE CEREBRALE ET LE TRAUMATISME CRANIOCEREBRAL

(30) Priorität: 09.12.1997 DE 19754573
(43) Veröffentlichungstag der Anmeldung: 17.01.2001
(73) Patentinhaber: Bayer HealthCare AG, 51368 Leverkusen (DE)
(72) Erfinder: HORVATH, Ervin, D-51373 Leverkusen (DE)
(86) Internationale Anmeldenummer: PCT/EP1998/007628
(87) Internationale Veröffentlichungsnummer: WO 1999/029346

(56) Entgegenhaltungen:
- EP-A- 0 352 613
- EP-A- 0 749 970
- DE-A- 4 135 474
- A.TAZI ET AL.: "Potentiation of behavioural effects of a calcium channel antagonist, nifedipine, by ipsapirone" BEHAVIOURAL PHARMACOLOGY, Bd. 3, Nr. 3, 1992, Seiten 269-273, XP002099065

## Beschreibung

Die Erfindung betrifft eine pharmazeutische Zusammensetzung umfassend wenigstens einen 5-HT_{1A}-Rezeptor Agonisten ausgewählt aus der Gruppe der Aminomethyl-Chromane und wenigstens einen Calciumkanal-Antagonisten sowie die Verwendung dieser pharmazeutischen Zusammensetzung zur Behandlung von Schlaganfall (Apoplexia cerebri) und Schädel-Hirn-Trauma.

Wie in der Literatur beschrieben, besitzen die Erkrankungen Schlaganfall (Apoplexia cerebri; ischemic stroke) und Schädel-Hirn-Trauma (traumatic brain injury; TBI) bestimmte gemeinsame pathomechanistische Elemente (z.B. hypoxisch-ischämischer Zelltod). Weiterhin ist bekannt, daß durch Hypoxie-Ischämie bedingten Sauerstoffund Energiemangel die zelluläre Ionenhomeostase zusammenbricht. Wesentliche Konsequenz dieses Geschehens ist die anoxysche Depolarisation (AD) der neuronalen Zellmembran in den betroffenen Hirngebieten, folgend der massiven Freisetzung von verschiedenen Neurotransmittern (z.B. Glutamat, Serotonin, usw.). Dem unkontrollierten Strömen verschiedener Ionen durch die Zellmembran folgt eine drastische und pathologische Erhöhung der intracellulären Calziumkonzentration, welche am Ende zum neuronalen Zelltod führt.

Es ist bekannt, daß 5-HT_{1A} Receptor Agonisten vom Typ der Aminomethyl-Chromane sowie bestimmte Calciumkanal-Antagonisten, insbesondere vom Typ der Dihydropyridine (welche den L-Typ Calciumkanal blockieren) neuroprotektive Eigenschaften in Tiermodellen des Schlaganfalls und Schädel-Hirn-Traumas aufweisen.

Die EP-A-0 004 650 beschreibt die Verbindung 1,4-Dihydro-2,6-dimethyl-4-(3'-nitrophenyl)-3,5-pyridindicarbonsäure-isopropylester-2-methoxyethylester (Nimodipin) und seine Wirkung auf cerebrale Durchblutungsstörungen, was ihren Einsatz bei der Einsatz bei der Behandlung cerebraler Insuffizienzen, insbesondere cerebraler Durchblutungsstörungen verschiedener Provenienz ermöglicht.

Die EP-A-0 352 613 beschreibt substituierte Aminomethyl-Tetraline sowie deren heterocyclische Analoga, insbesondere Aminomethyl-Chromane und deren Wirkung zur Bekämpfung von Krankheiten, die durch Störungen des serotoninergen Systems, insbesondere bei Involvierung von Receptoren, die eine hohe Affinität zu 5-Hydroxytryptamin besitzen (5-HT_{1A}-Typ) gekennzeichnet sind.

Die EP-A-0 540 914 beschreibt 2-Aminomethyl-Chromane und deren Verwendung als Wirkstoffe in Arzneimitteln zur Behandlung von Erkrankungen des zentralen Nervensystems (ZNS).

Die EP-A-0 749 970 beschreibt in diesem Zusammenhang Benzisothiazolyl-substituierte 2-Aminomethyl-Chromane und deren Wirksamkeit bei der Behandlung von Schädigungen infolge cerebralen Infarkten.

Die DE-OS 195 43 476 beschreibt die Verwendung von 2-Aminomethyl-Chromanen bei der Behandlung von Schädigungen in Folge Schädel-Hirn-Trauma.

Es war nun eine Aufgabe der vorliegenden Erfindung eine pharmazeutische Zusammensetzung zur Verfügung zu stellen, die bei der Behandlung von Schlaganfall und Schädel-Hirn-Trauma eine verbesserte Wirkung aufweist.

Diese Aufgabe wurde gelöst durch eine pharmazeutische Zusammensetzung umfassend wenigstens einen 5-HT_{1A}-Agonisten und wenigstens einen Calciumkanal-Antagonisten.

5-HT_{1A}-Agonisten im Sinne der Erfindung sind Verbindungen, die die Forskolin-stimulierte Adenylatcyclase-Aktivität an hippocampalen Zellmembranen mit einem IC₅₀-Wert von weniger als 10.000 nM, bevorzugt weniger als 1.000 nM, besonders bevorzugt weniger als 100 nM, ganz besonders bevorzugt weniger als 10 nM inhibieren.

Kalziumkanalantagonisten im Sinne der Erfindung sind Verbindungen, die die KCl-induzierte Kontraktion isolierter Aortaringe des Kaninchens mit einem IC₅₀-Wert von weniger als 10.000 nM, bevorzugt weniger als 1.000 nM, besonders bevorzugt weniger als 100 nM, ganz besonders bevorzugt weniger als 10 nM inhibieren.

Im Rahmen der Erfindung geeigente 5-HT_{1A}-Agonisten sind beispielsweise Roxindolmesylat (Drugs of the Future, 1995, 20 (12), Seiten 1228-1232);
N4-Imidoethyl-Derivate von 1-(2,3-Dihydro-1,4-benzodioxin-5-yl)piperazin (J. Med. Chem. 1995, 38, Seiten 4303-4308);
5-[3-[(2S)-(1,4-Benzodioxan-2-ylmethyl)amino]propoxy]-1,3-benzodioxol Hydrochlorid (Society for Neuroscience Abstracts 19 (1-3), Seite 1243 (1993));
die in der EP-A-0 648 767 offenbarten Piperidin- und Piperazin-Derivate;
(+)-R-2-Cyano-N,N-dipropyl-8-amino-6,7,8,9-tetrahydro-3H-benz(e)indol (The Journal of Pharmacology and Experimental Therapeutics, 271(2), 1994, Seiten 875-883);
(-)-4R-6-Acetyl-4-(di-n-propylamino)-1,3,4,5-tetrahydrobenz[c,d]indol (The Journal of Pharmacology and Experimental Therapeutics, 270 (3), 1994, Seiten 1270-1281);
die Enantiomeren von 8-Thiomethyl-2-(di-n-propylamino)tetralin (Drug Development Research, 34, 1995, Seiten 66-85);
ω-(Tetralin-1-yl)-n-alkylamin-Derivate (J. Med. Chem. 1996, 39, Seiten 176-182);
Flesinoxan Hydrochlorid;
4-(2-Heteroarylethyl)-1-arylpiperazine (Arzneimittel-Forschung 47(1), 1997, Seiten 239-243);
1-[2-(2-Naphthyl)ethyl]-4-[3-(trifluoromethyl)phenyl]-1,2,3,6-tetrahydropyridin Hydrochlorid (Neuroscience, 1993, Seiten 1-13);
die im Annual Drug Report 1997 auf Seite 23 unter Referenz 240964 beschriebenen Benzoxazepin-Derivate;
die im US-Patent 5,602,128 beschriebenen N-Heterocycloalkyl carboxamide;
die in der WO-97/30050 beschriebenen Heterocyclyl-ergolin-Derivate;
6-Methoxy-4-(di-n-propylamino)-1,3,4,5-tetrahydrobenz[c,d]indol Hydrochlorid (Brain 5-HT_{1A} [HTA] receptors, Ellis Horwood und VCH Verlagsgesellschaft, 1987, Kapitel 9);
die in der EP-A-0 496 222 beschriebenen Verbindungen;
4[N-(5-Methoxy-chroman-3-yl)N-propylamino]butyl-8-azaspiro-(4,5)-decan-7,9-dion und dessen Enantiomere (The Journal of Pharmacology and Experimental Therapeutics, 264 (2), 1993, Seiten 863-872);
1-[2-(2-thenoylamino)ethyl]-4[1-(7-methoxy naphthyl)]piperzin (The Journal of Pharmacology and Experimental Therapeutics, 262 (2), 1992, Seiten 451-463);
4-(Benzodioxan-5-yl)1-(indan-2-yl)piperazin (The Journal of Pharmacology and Experimental Therapeutics, 268 (1), 1994, Seiten 337-352);
1-[2-(4-Fluorobenzoylamino)ethyl]-4-(7-methoxy naphthyl)piperazin Hydrochlorid (Drug Development Research 26, 1992, Seiten 21-48).

Bevorzugte 5-HT_{1A}-Agonisten sind ausgewählt aus der Gruppe der 2-Aminomethyl-Chromane, insbesondere aus der Gruppe der Verbindungen der nachfolgenden Formel (I) 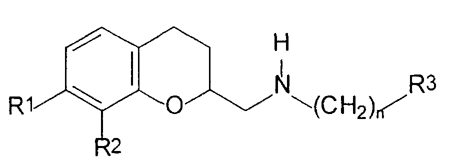 worin
- R¹: für Wasserstoff steht,
- R²: für Wasserstoff, Hydroxy, einen Carbamoyl-Rest oder einen Rest der Formel -OCH₃, -OCH₂CH₃, -OCH(CH₃)₂ oder -OCH₂C(CH₃)₂-Cl steht, oder
- R¹ und R²: gemeinsam einen Rest der Formel 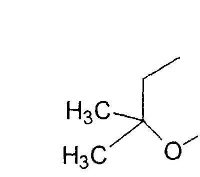
bilden,
- R³: für Cyclopentyl, Cyclohexyl, Cycloheptyl, Cyclooctyl oder oder den nachfolgenden, als o-Benzosulfimidyl bezeichneten Rest: 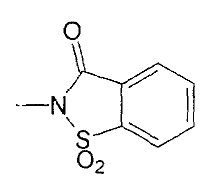
steht und n ausgewählt ist aus 1, 2, 3, 4 oder 5
sowie deren optische Isomeren und pharmazeutisch annehmbaren Salze.

Für den Fall, daß R³ den Benzosulfimidyl-Rest repräsentiert, ist n vorzugsweise 4 oder 5.

Besonders bevorzugte Aminomethyl-Chromane der Formel (I) sind dadurch gekennzeichnet, daß
R¹ = Wasserstoff,
R² = Wasserstoff, -OCH₃, OCH(CH₃)₂ oder -OCH₂C(CH₃)₂-Cl, oder
R¹ und R² gemeinsam einen Rest der Formel 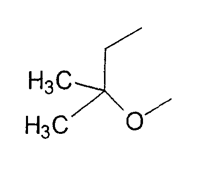 bilden,
R³ = o-Benzosulfimidyl und
n = 4 ist.

Ganz besonders bevorzugte Aminomethyl-Chromane sind ausgewählt aus der Gruppe der Verbindungen der Formel (I), wobei
R¹ = Wasserstoff,
R² = Wasserstoff oder -OCH₃,
R³ = o-Benzosulfimidyl und
n = 4 ist;
oder
R¹ = Wasserstoff,
R² = -OCH(CH₃)₂,
R³ = o-Benzosulfimidyl und
n = 4 ist;
oder
R¹ = Wasserstoff,
R² = -OCH₂C(CH₃)₂Cl,
R³ = o-Benzosulfimidyl und
n = 4 ist;
oder
R¹ und R² gemeinsam einen Rest der Formel 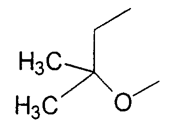 bilden,
R³ = o-Benzosulfimidyl und n = 4 ist.

Im Rahmen der vorliegenden Erfindung können die Aminomethyl-Chromane der Formel (I) auch als ihre physiologisch unbedenklichen Salze vorliegen. Physiologisch unbedenkliche Salze der substituierten 2-Aminomethyl-Chromane können Salze der erfindungsgemäßen Stoffe mit anorganischen oder organischen Säuren, insbesondere mit Mineralsäuren, Carbonsäuren oder Sulfonsäuren sein. Besonders bevorzugt sind z.B. Salze mit Chlorwasserstoffsäure, Bromwasserstoffsäure. Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Ferner können im Rahmen der vorliegenden Erfindung die Verbindungen der Formel (I) auch in verschiedenen stereoisomeren Formen vorliegen. In den erfindungsgemäßen pharmazeutischen Zusammensetzungen können die Verbindungen der Formel (I) in Form ihres Racemates oder in Form des (-)-Enantiomers oder des (+)-Enantiomers eingesetzt werden. Bevorzugt sind jedoch die (-)-Enantiomere der Verbindungen der Formel (I).

Besonders bevorzugt im Rahmen der vorliegenden Erfindung sind Verbindungen der Formel (I), in denen der Substituent R² für Wasserstoff oder Methoxy steht. Ganz besonders bevorzugt sind die (-)-Enantiomere der Verbindung der Formel (I), insbesondere für den Fall, daß der Substituent R¹ und R² für Wasserstoff steht, sowie jeweils deren physiologisch unbedenklichen Salze.

Zur Herstellung der substituierten Aminomethyl-Chromane der Formel (I) wird auf die EP-A-0 352 613, die EP-A-0 540 914 und die EP-A-0 749 970 verwiesen.

Im Rahmen der Erfindung bevorzugt sind Calciumkanal-Antagonisten, die aus der Gruppe der Dihydropyridin-Calciumkanal-Antagonisten ausgewählt sind. Besonders bevorzugte Calciumkanal-Antagonisten sind Dihydropyridine der Formel (II) 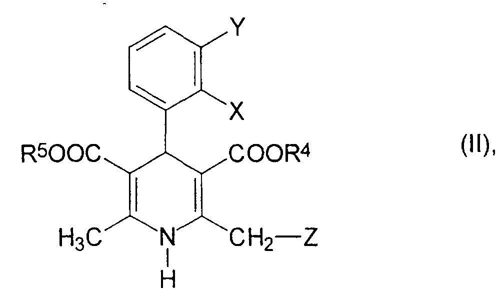 worin
- X und Y: gleich oder verschieden sind und für Wasserstoff, Nitro (-NO₂) oder Halc gen stehen, oder
- X und Y: gemeinsam mit dem Phenylring einen Benzoxadiazolylrest bilden,
- R⁴ und R⁵: gleich oder verschieden sind und für geradkettiges oder verzweigtes Alkyl mit bis zu 6 Kohlenstoffatomen stehen, das durch Alkoxy mit bis zu 4 Kohlenstoffatomen, eine N-Benzyl-N-methyl-amino-Gruppe oder eine 4-(Diphenylmethyl)-piperazino-Gruppe substituiert sein kann, oder
für eine Gruppe der Formel 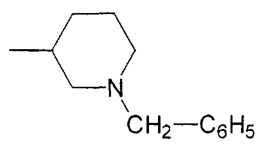 stehen,
und
- Z: für Wasserstoff oder für die Gruppe -OCH₂CH₂-NH₂ steht,
oder deren pharmazeutisch akezeptablen Salze.

Die Halogen-Substituenten in den Calciumkanal-Antagonisten der Formel (II) sind ausgewählt aus der Gruppe bestehend aus Fluor, Chlor, Brom und Iod.

Ferner sind bevorzugt die Calciumkanal-Antagonisten (INN) Barnidipin, Benidipin, Efonidipin, Lacidipine, Nilvadipin, Anipamil, Aranidipin, Azelnidipin, Cilnidipin, Elgodipin, Furnidipin, Iganidipin, Lemildipin, Lercanidipin, Oxodipin, Amlodipin, Clevidipin, Diperdipin, Felodipin, Isradipin, Lacidipin, Lercanidipin, Manidipin, Nicardipin, Nifedipin, Nimodipin, Nisoldipin, Nitrendipin, Trombodipin, Watanidipin hydrochloride und Pranidipin oder ihre pharmazeutisch akzeptablen Salze, sofern sie existieren.

Die Dihydropyridine der Formel (II) und deren Herstellung ist aus der EP-A-0 004 650 und der darin zitierten weiteren Literatur bekannt.

Besonders bevorzugt im Rahmen der vorliegenden Erfindung sind die Dihydropyridine der allgemeinen Formel (II), in welcher
- X und Y: gleich oder verschieden sind und für Wasserstoff, Nitro oder Chlor stehen,
- R⁴ und R⁵: gleich oder verschieden sind und für geradkettiges oder verzweigtes Alkyl mit bis zu 4 Kohlenstoffatomen stehen, das durch Methoxy substituiert sein kann und
- Z: für Wasserstoff steht.

Ganz besonders bevorzugt ist (RS)-1,4-Dihydro-2,6-dimethyl-4-(3-nitrophenyl)-3,5-pyridindicarbonsäure-isopropylester-2-methoxyethylester (INN: Nimodipin), in welcher in obiger Formel (II) X = H, Y = -NO₂, Z = H, R⁴ = Isopropyl und R⁵ = Methyloxyethyl ist.

Ganz besonders bevorzugt im Rahmen der Erfindung ist eine pharmazeutische Zusammensetzung umfassend das (-)-Enantiomer der Verbindung (I), in der R¹ und R² Wasserstoff ist R³ = o-Benzosulfimidyl und n = 4 und Nimodipin als Verbindung der Formel (II).

Die 5-HT_{1A}-Agonisten, insbesondere die Aminomethyl-Chromane der allgemeinen Formel (I) und Calciumkanal-Antagonisten, insbesondere der allgemeinen Formel (II) zeigen in beiden Tiermodellen, dem permanenten fokalen cerebralen Ischämiemodell der Ratte (middle cerebral artery occlusion; MCA-O Modell) und dem akuten subduralen Hämatom der Ratte (SDH Modell) gute neuroprotektive Wirkung.

Die Wirkung der Calciumkanal-Antagonisten, insbesondere der allgemeinen Formel (II), beschränkt sich auf das Rattenmodell des subduralen Hämatoms (SDH); im Modell der permanenten fokalen cerebralen Ischämie (MCA-O) zeigten diese Substanzen nur eine schwache Wirkung. Die erfindungsgemäße Kombination beider Substanzen ergab überraschenderweise eine synergistische Neuroprotektion im Modell der permanenten fokalen cerebralen Ischämie (MCA-O; Bederson et al., Stroke 1986, 17, 472).

Die Kombination von 5-HT_{1A}-Agonisten mit Calciumkanal-Antagonisten, insbesondere der Verbindungen der Formeln (I) und (II) bewirken bei einer nach Schlaganfall oder Schädel-Hirn-Trauma erfolgenden therapeutischen Behandlung eine deutliche Aufhebung des Absterbens von Neuronen und Gliazellen im Hirngewebe.

Die Behandlung von Schlaganfall und Schädel-Hirn-Trauma mit der Kombination aus 5-HT_{1A}-Agonisten und Calciumkanal-Antagonisten und insbesondere der Wirkstoffe der allgemeinen Formeln (I) und (II) stellt ein neues Behandlungsprinzip dar. Die erfindungsgemäße Wirkstoffkombination kann dementsprechend zur Behandlung von Schlaganfall und Schädel-Hirn-Trauma verwendet werden, bzw. zur Herstellung von Arzneimitteln zur Behandlung von Schlaganfall und Schädel-Hirn-Trauma.

Die erfindungsgemäße Wirkstoffkombination kann in bekannter Weise in die üblichen Formulierungen überführt werden, wie Tabletten, Dragees, Pillen, Granulate, Aerosole, Sirupe, Emulsionen, Suspension und Lösungen, unter Verwendung inerter, nicht toxischer, pharmazeutisch geeigneter Trägerstoffe oder Lösungsmittel. Hierbei soll die therapeutisch wirksame Wirkstoffkombination jeweils in einer Konzentration von etwa 0,1 bis 95 Gew.-%, bevorzugt von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe erwähnt seien beispielsweise Wasser, nicht-toxische organische Lösungsmittel, wie Paraffine (z.B. Erdölfraktionen), pflanzliche Öle (z.B. Erdnuß/-Sesamöl), Alkohole (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den oben genannten Hilfsstoffen mit verschiedenen Geschmacksaufbesserern oder Farbstoffen versetzt werden. Für den Fall der parenteralen Anwendung können Lösungen der Wirkstoffe unter Verwendung geeigneter flüssiger Trägermaterialien eingesetzt werden.

Die Erfindung betrifft ferner ein Kit umfassend in getrennter Form eine pharmazeutische Zusammensetzung enthaltend den 5-HT_{1A}-Agonisten, wie oben-beschrieben und eine pharmazeutische Zusammensetzung enthaltend den Calciumkanal-Antagonisten wie oben beschrieben. Die Zurverfügungstellung der erfindungsgemäßen Zusammensetzung in Form ihrer getrennten Komponenten kann insbesondere dann von Interesse sein, wenn die Wirkstoffe oder ihre Lösungen nicht miteinander kompatibel sind und dementsprechend getrennt aufbewahrt und/oder verabreicht werden.

Im allgemeinen hat es sich bei intravenöser Applikation als vorteilhaft erwiesen, den 5-HT_{1A}-Agonisten in Mengen von etwa 0,001 µg/kg/h bis 1 mg/kg/h (µg oder mg pro kg Körpergewicht pro Stunde), vorzugsweise etwa 0,01 µg/kg/h bis 0,1 mg/kg/h und den Calciumkanal-Antagonisten in Mengen von etwa 0,1 µg/kg/h bis 10 mg/kg/h, vorzugsweise 1 µg/kg/h bis 5 mg/kg/h zur Erzielung wirksamer Ergebnisse zu verabreichen. Die Verabreichung kann jeweils in Form von Einzelgaben erfolgen.

Bei oraler Applikation beträgt die Tagesdosis des 5-HT_{1A}-Agonisten 0,001 bis 10 mg/kg vorzugsweise 0,01 bis 1 mg/kg, die des Calciumkanal-Antagonisten 0,001 bis 100 mg/kg, vorzugsweise 0,01 bis 10 mg/kg.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Die Erfindung wird anhand des nachfolgenden Beispiels näher erläutert.

### Beispiel:

### I. Inhibition der Forskolin-stimulierten Adenylatcyclase-Aktivität an hippocampalen Zellmembranen durch 5-HT_{1A}-Agonisten

5-HT_{1A} Rezeptor gekoppelte Adenylatcyclase-Aktivität wurde an hippocampalen Zellmembranen der Ratte mit der Methodik von De Vivo und Maayani (J. Pharmacol. Exp. Ther. 1986, 238, 248-253.) gemessen.

Der Hippocampus wurde in 25 Volumen 0,3 M Sucroselösung (ergänzt mit 1mM EGTA, 5 mM EDTA, 5 mM Dithiothreitol und 20 mM Tris-HCl; pH 7,4 bei 25°C) in einem motorgetriebenen Potter-Elvehjem-Homogenisator zerkleinert. Das Homogenat wurde 10 min lang bei 1.000 g abzentrifugiert, danach der Überstand 10 min bei 39.000 g nochmals zentrifugiert. Das Pellet wurde im Homogenisationspuffer aufgenommen. Aliquots mit einem Proteingehalt von 1 mg/ml (gemessen nach Bradford Anal. Biochem. 1976, 72, 248-254.) wurden bei -140°C bis zur weiteren Verarbeitung aufbewahrt. Vor dem Assay wurde das aufgetaute Homogenat mit dem Potter-Elvehjem-Homogenisator nochmals homogenisiert. 50 µl Homogenat (mit ca. 50 µg Proteingehalt) wurden der vorgewärmten (5 min bei 30°C) Inkubationsmischung (100 mM NaCl, 2mM Magnesium Acetate 0,2 mM ATP, 1 mM cAMP, 0,01 mM GTP, 0,01 mM Forskolin, 80 mM Tris-HCl, 5 mM Creatine Phosphate, 0,8 U/µl Creatine Phosphokinase, 0,01 mM IBMX, 1 - 2 µCi α-[³²P]ATP) zupipettiert und so die Inkubation (10 min bei 30°C) gestartet.

Zur Messung der Adenylatcyclase-Aktivität wurde die Menge des gebildeten [³²P]cAMP nach Salomon (Adv. Cyclic Nucleotide Res. 1979, 10, 35-55.) bestimmt.

Beispielsweise inhibiert das (-)-Enantiomere der Verbindung der Formel (I) mit R¹ = R² = H, R³ = o-Benzosulfimidyl und n = 4 die Forskolin-stimulierte Adenylatcyclase Aktivität an Hippocampusmembranen der Ratte mit einem IC₅₀-Wert von 1,6 nM.

### II. Inhibition der KCl-induzierten Kontraktion isolierter Aortaringe des Kaninchens durch Kalziumkanalantagonisten

Im Allgemeinen heben Kalziumkanalantagonisten die KCl-induzierte Kontraktion isolierter Aortaringe des Kaninchens auf (vgl. Arzneim.-Forsch./Drug Res. 1992, 42, 795-797; J. Pharmacol. Exp.Ther. 1953, 108,129-143.).

Kaninchen beiderlei Geschlechts werden schmerzlos betäubt und entblutet. Der thorakale Teil der Aorta descendens wird ohne Verletzung der Endothelschicht freipräpariert und auf ca. 2 mm breite spiraienförmige Streifensegmente geschnitten und einzeln unter einer Anfangsbelastung von ca. 2 g in 10ml Carbogen-begaste Krebs-Bicarbonat-Pufferlösung (37°C) verbracht.

Kontraktionen werden isometrisch erfaßt und ausgewertet.

Die funktionelle Integrität des Endotheliums wird durch Noradrenalin (1 x 10⁻⁸ mol/l) induzierte Kontraktionen und durch Acetylcholin (1 x 10⁻⁸ - 1 x 10⁻⁵ mol/l) hervorgerufene Relaxation getestet. Nach einer Auswasch- und Ruhephase sowie Erreichen eines stabilen Basalwertes (ca. nach 2 Stunden) wird mit Zugabe von 0,25 ml KCl, bei einer Endkonzenration von 1 x 10⁻² mol/l eine submaximale (60-80%) Kontraktion der Aortastreifen ausgelöst. Die Testsubstanz wird während der Plateauphase der KCl induzierten Kontraktion in die Bäder appliziert (in steigender Dosierungen: 1 x 10⁻¹² - 1 x 10⁻⁵ mol/l Endkonzentration) und die Wirkung auf den kontrahierten Gefäßstreifen gemessen.

Individuelle EC₅₀-Werte für jeden Aortastreifen werden durch die lineare Regression aus der Auftragung des Prozentsatzes der Substanz-induzierten Relaxation gegen den Logarithmus der Dosierungen dieser Testsubstanz berechnet. Aus diesem wird der Mittelwert ± Standardabweichung des EC₅₀-Wertes ermittelt.

Der Kalziumkanal Antagonist Nifedipin inhibiert beispielsweise die KCl-induzierte Kontraktion der Kaninchen-Aortenringe mit einem EC₅₀-Wert von 3,58 ± 1.67 nM. Unter vergleichbarer Versuchsanordnung ist der EC₅₀-Wert für Nimodipin 2,9 - 4,3 nM.

### III. Bestimmung der Neuroprotektion im Rattenmodell des subduralen Hämatoms (SDH)

Dieses Modell ist in Neurosurg. 1990, 27, 433-9 beschrieben.

Unter Isofluran Anästhesie wird die Kopfhaut entlang der Saggitalnaht geöffnet. Über dem motorischen Cortex wird der Schädelknochen punktförmig durchbohrt, die darunterliegende Hirnhaut eröffnet und ein vorangefertigter spezieller Plastikkatheter eingeführt. Dieser Katheter wird mit Gewebekleber an der Schädeldecke befestigt. Aus der Schwanzvene entnommenes Eigenblut (200 ul) wird über eine Kanüle durch den fixierten Plastikkatheter unter die Hirnhaut injiziert. Nach Verschluß des Katheters werden die Tiere in ihren Heimkäfig zurückgesetzt.

Im Verlauf der folgenden Stunden entwickelt sich unterhalb des Hämatoms ein Infarkt, dessen Ausdehnung histologisch quantifiziert wird. Die Bestimmung der Infarktgröße erfolgt wie beim Modellb der Permanenten Focalen Ischämie bei der Ratte (MCA-O) beschrieben.

Die Tiere wurden im allgemeinen direkt nach der chirurgischen Induktion des Hämatoms oder zu verschiedenen Zeitpunkten danach behandelt. Die Substanzapplikation könnte erfolgen nach unterschiedlichen zeitlichen Schemata und über unterschiedliche Applikationswege (i.v., i.p. usw).

### IV. Bestimmung der Neuroprotektion im Rattenmodell der permanenten fokalen cerebralen Ischämie (MCA-O)

### 1. Permanenter Verschluß der Arteria cerebri media bei der Ratte

Unter allgemeiner Narkose (Isofluran) wird in der Mitte zwischen Auge und Ohr ein Hautschnitt gelegt und unter Durchtrennung der darunterliegenden Muskelpartien ein Zugang zum Schädel im Bereich des Foramen ovale geschaffen. Etwa 1 mm rostrodorsal dieser Öffnung wird der Schädelkalotte durchgebohrt, die darunterliegenden Hirnhäute eröffnet, und durch Elektrokoagulation die Arteria cerebri media und deren Nebenäste permanent verschlossen. Daraufhin wird die Kaumuskulatur in ihre ursprüngliche Lage gebracht. Die Muskel- und Hautwunde wird chirurgisch verschlossen und versorgt. Nach dem Aufwachen aus der Narkose werden die Tiere in ihren Heimkäfig zurückgesetzt. Im Verlauf der folgenden Stunden entwickelt sich im Versorgungsgebiet der Arteria cerebri media ein Infarkt, dessen Ausdehnung histologisch quantifiziert wird.

### 2. Quantifizierung der Infarktgröße

Nach 7 Tagen Überlebenszeit werden die Tiere getötet, das Gehirn entnommen und in auf -30°C gekühltem 2-Methylbutan eingefroren. Mit Hilfe eines Kryostatmikrotoms werden 20 µm dicke Schnitte in 500 µm Abstand im Infarktbereich hergestellt. Nach der Cresylechtviolett-Färbung der fixierten Gehirnschnitte ist das Infarktgebiet vom nicht betroffenen Gewebe deutlich zu unterscheiden. Das Volumen des geschädigten cortikalen (subcortikalen) Hirngewebe (Infarkt) wird duch planimetrische Messung der Infarktoberfläche/Schnitt unter Berücksichtigung von Schnittzahl- und Abstand mit Hilfe eines computerisierten Bildanalysesystems berechnet.

### 3. Arzneistoffbehandlung

Die Tiere wurden direkt nach dem chirurgischen Verschluß der Arteria cerebri media behandelt. Beide Wirkstoffe wurden in deren Placebolösung aufgenommen und in einem Volumen von insgesamt 4 ml/kg Körpergewicht/Stunde als i.v. Infusion über 4 Stunden allein oder in Kombination infundiert. Dosierung des Aminomethyl-chroman beträgt 0,0001 mg/kg Körpergewicht/Stunde des Calciumkanal-Antagonisten 0,003 mg/kg Körpergewicht/Stunde.

Das Ergebnis der Untersuchung ist beispielhaft in der nachfolgenden Tabelle gezeigt.

**Tabelle**

| Wirkstoff | Dosierung | Infarktvolumen im cerebralen Cortex (Mittelwert ± S.E.M; mm³) |
|---|---|---|
| Placebo | 4 ml/kg/Std | 146,69 ± 6,77 |
| 5-HT_{1A}-Agonist* | 0,0001 1 mg/kg/Std | 104,79 ± 7,63 |
| Nimodipin | 0,003 mg/kg/Std | 120,87 ± 8,79 |
| 5-HT_{1A}-Agonist* + Nimodipin | 0,0001 mg/kg/Std + 0,003 mg/kg/Std | 70,69 ± 13,73** |

| | | |
|---|---|---|
| * (-)-Enantiomeres der Verbindung der Formel (I) mit R¹ = R² = H, R³ = o-Benzosulfimidyl und n = 4 | | |
| ** = p ≤ 0.005 (LSD-Test) | | |

## Patentansprüche

1. Pharmazeutische Zusammensetzung umfassend wenigstens einen 5-HT_{1A}-Agonisten ausgewählt aus der Gruppe der Aminomethyl-Chromane und wenigstens einen Calciumkanal-Antagonisten.

2. Pharmazeutische Zusammensetzung gemäß Anspruch 1, **dadurch gekennzeichnet, daß** die Aminomethyl-Chromane ausgewählt sind aus der Gruppe der Verbindungen der nachfolgenden Formel (I) 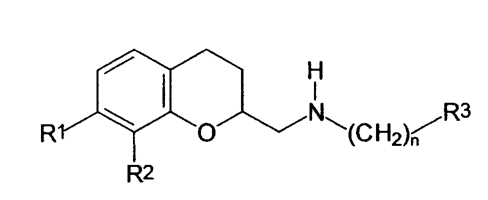 worin
R¹ für Wasserstoff steht,
R² für Wasserstoff, Hydroxy, einen Carbamoyl-Rest oder einen Rest der Formel -OCH₃, -OCH₂CH₃, -OCH(CH₃)₂ oder -OCH₂C(CH₃)₂-Cl steht, oder
R¹ und R² gemeinsam einen Rest der Formel 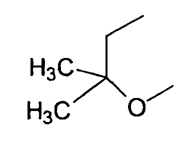
bilden,
R³ für Cyclopentyl, Cyclohexyl, Cycloheptyl, Cyclooctyl oder o-Benzosulfimidyl steht und n ausgewählt ist aus 1, 2, 3, 4 oder 5
sowie deren optische Isomeren und pharmazeutisch annehmbaren Salze.

3. Pharmazeutische Zusammensetzung nach Anspruch 2, **dadurch gekennzeichnet, daß** das Aminomethyl-Chroman eine Verbindung ist, worin
R¹ = Wasserstoff,
R² = Wasserstoff, -OCH₃, -OCH(CH₃)₂, oder -OCH₂C(CH₃)₂-Cl,
oder R¹ und R² zusammen einen Rest der Formel 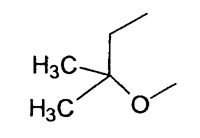 bilden,
R³ = o-Benzosulfimidyl und
n = 4 ist.

4. Pharmazeutische Zusammensetzung gemäß Anspruch 3, **dadurch gekennzeichnet, daß** das Aminomethyl-Chroman ausgewählt ist aus der Gruppe der Verbindung, worin
R¹ = Wasserstoff und R² = Wasserstoff oder -OCH₃ ist;
oder
R¹ = Wasserstoff und R² = -OCH(CH₃)₂ ist;
oder
R¹ = Wasserstoff und R² = -O-CH₂C(CH₃)₂Cl;
oder
R¹ und R² gemeinsam einen Rest der Formel 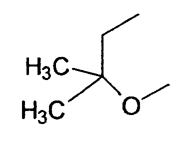 bilden.

5. Pharmazeutische Zusammensetzung nach einem oder mehreren der Ansprüche 2 bis 4, **dadurch gekennzeichnet, daß** das Aminomethyl-Chroman die (-)-Enantiomer-Konfiguration aufweist.

6. Pharmazeutische Zusammensetzung nach Anspruch 1, **dadurch gekennzeichnet, daß** der Calciumkanal-Antagonist ausgewählt ist aus der Gruppe der Dihydropyridin-Calciumkanal-Antagonisten.

7. Pharmazeutische Zusammensetzung nach Anspruch 6, **dadurch gekennzeichnet, daß** der Calciumkanal-Antagonist ein Dihydropyridin der nachfolgenden Formel (II) 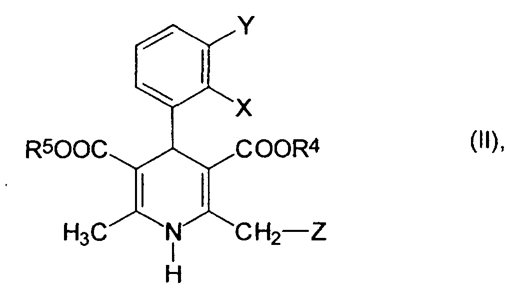 ist, worin
X und Y gleich oder verschieden sind und für Wasserstoff, Nitro (-NO₂) oder Halogen stehen, oder
X und Y gemeinsam mit dem Phenylring einen Benzoxadiazolylrest bilden,
R⁴ und R⁵ gleich oder verschieden sind und für geradkettiges oder verzweigtes Alkyl mit bis zu 6 Kohlenstoffatomen stehen, das durch Alkoxy mit bis zu 4 Kohlenstoffatomen, eine N-Benzyl-N-methyl-amino-Gruppe oder eine 4-(Diphenylmethyl)-piperazino-Gruppe substituiert sein kann, oder
für eine Gruppe der Formel 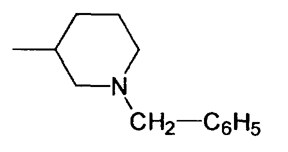 stehen,
und
Z für Wasserstoff oder für die Gruppe -O-CH₂CH₂-NH₂ steht,
oder deren pharmazeutisch akzeptablen Salze.

8. Pharmazeutische Zusammensetzung nach Anspruch 7, **dadurch gekennzeichnet, daß** im Dihydropyridin der Formel (II)
X und Y gleich oder verschieden sind und für Wasserstoff, Nitro oder Chlor stehen,
R⁴ und R⁵ gleich oder verschieden sind und für geradkettiges oder verzweigtes Alkyl mit bis zu 4 Kohlenstoffatomen stehen, das durch Methoxy substituiert sein kann und
Z für Wasserstoff steht.

9. Pharmazeutische Zusammensetzung nach Anspruch 7, **dadurch gekennzeichnet, daß** im Dihydropyridin der Formel (II)
X = H, Y = NO₂, Z = H, R⁴ = Isopropyl und R⁵ = Methyloxyethyl ist.

10. Verwendung der pharmazeutischen Zusammensetzung gemäß irgendeinem der Ansprüche 1 bis 9 zur Herstellung eines Medikaments zur Behandlung von Schlaganfall (Apoplexia cerebri) und Schädel-Hirn-Trauma.

11. Kit umfassend in getrennter Form eine pharmazeutische Zusammensetzung enthaltend den 5-HT_{1A}-Agonisten ausgewählt aus der Gruppe der Aminomethyl-Chromane wie in einem oder mehreren der Patentansprüche 1 bis 5 definiert und eine pharmazeutische Zusammensetzung enthaltend den Calciumkanal-Antagonisten wie in einem oder mehreren der Patentansprüche 6 bis 9 definiert.

## Claims

1. Pharmaceutical composition comprising at least one 5-HT_{1A} agonist selected from the aminomethyl-chromans group and at least one calcium channel antagonist.

2. Pharmaceutical composition according to Claim 1, **characterized in that** the aminomethyl-chromans are selected from the group of compounds of the following formula (I) 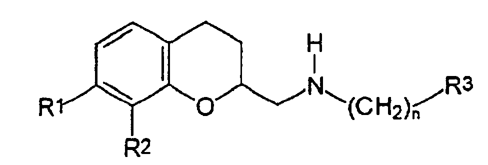 in which
R¹ represents hydrogen,
R² represents hydrogen, hydroxyl, a carbamoyl radical or a radical of the formula -OCH₃, -OCH₂CH₃, -OCH(CH₃)₂ or -OCH₂C(CH₃)₂-Cl, or
R¹ and R² together form a radical of the formula 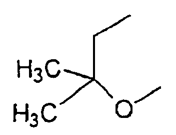
R³ represents cyclopentyl, cyclohexyl, cycloheptyl, cyclooctyl or obenzosulphimidyl and n is selected from 1, 2, 3, 4 or 5
and their optical isomers and pharmaceutically acceptable salts.

3. Pharmaceutical composition according to Claim 2, **characterized in that** the aminomethyl-chroman is a compound, in which
R¹ = hydrogen,
R² = hydrogen, -OCH₃, OCH(CH₃)₂ or -OCH₂C(CH₃)₂-Cl, or
R¹ and R² together form a radical of the formula 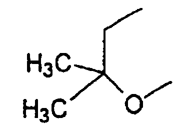 R³ = o-benzosulphimidyl and
n=4.

4. Pharmaceutical composition according to Claim 3, **characterized in that** the aminomethyl-chroman is selected from the group of compound, in which
R¹ = hydrogen and R² = hydrogen or -OCH₃;
or
R¹ = hydrogen and R² = -OCH(CH₃)₂;
or
R¹ = hydrogen and R² = -O-CH₂C(CH₃)₂Cl;
or
R¹ and R² together form a radical of the formula

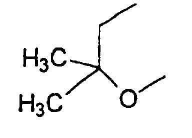

5. Pharmaceutical composition according to one or more of Claims 2 to 4, **characterized in that** the aminomethyl-chroman has the (-)-enantiomer configuration.

6. Pharmaceutical composition according to Claim 1, **characterized in that** the calcium channel antagonist is selected from the dihydropyridine calcium channel antagonists group.

7. Pharmaceutical composition according to Claim 6, **characterized in that** the calcium channel antagonist is a dihydropyridine of the following formula (II) 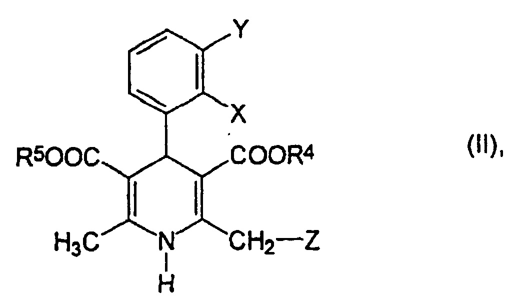 in which
X and Y are identical or different and represent hydrogen, nitro (-NO₂) or halogen, or
X and Y, together with the phenyl ring, form a benzoxadiazolyl radical,
R⁴ and R⁵ are identical or different and represent straight-chain or branched alkyl having up to 6 carbon atoms, which can be substituted by alkoxy having up to 4 carbon atoms, an N-benzyl-N-methyl-amino group or a 4-(diphenylmethyl)-piperazino group, or
represent a group of the formula 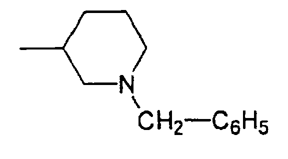 and
Z represents hydrogen or the group -O-CH₂CH₂-NH₂,
or their pharmaceutically acceptable salts.

8. Pharmaceutical composition according to Claim 7, **characterized in that**, in the dihydropyridine of the formula (II),
X and Y are identical or different and represent hydrogen, nitro or chlorine,
R⁴ and R⁵ are identical or different and represent straight-chain or branched alkyl having up to 4 carbon atoms, which can be substituted by methoxy and
Z represents hydrogen.

9. Pharmaceutical composition according to Claim 7, **characterized in that**, in the dihydropyridine of the formula (II),
X = H, Y = NO₂, Z = H, R⁴ = isopropyl and R⁵ = methyloxyethyl.

10. Use of the pharmaceutical composition according to any of Claims 1 to 9 for the production of a medicament for treating stroke (cerebral apoplexia) and craniocerebral trauma.

11. Kit including, in separate form, a pharmaceutical composition comprising the 5-HT_{1A} agonist selected from the aminomethyl-chromans group as defined in one or more of Patent Claims 1 to 5 and a pharmaceutical composition comprising the calcium channel antagonist as defined in one or more of Patent Claims 6 to 9.

## Revendications

1. Composition pharmaceutique, comprenant au moins un agoniste du récepteur 5-HT_{1A}- choisi dans le groupe des aminométhylchromanes et au moins un antagoniste des canaux calcium.

2. Composition pharmaceutique suivant la revendication 1, **caractérisée en ce que** les aminométhylchromanes sont choisis dans le groupe des composés de formule (I) suivante 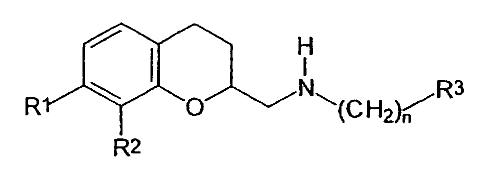 dans laquelle
R¹ représente l'hydrogène,
R² représente l'hydrogène, un groupe hydroxy, un reste carbamoyle ou un reste de formule -OCH₃, -OCH₂CH₃, -OCH(CH₃)₂ ou -OCH₂C(CH₃)₂-Cl, ou bien
R¹ et R² forment ensemble un reste de formule 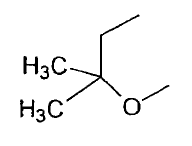
R³ est un reste cyclopentyle, cyclohexyle, cycloheptyle, cyclooctyle ou o-benzosulfimidyle et n a une valeur choisie de 1, 2, 3, 4 ou 5,
ainsi que leurs isomères optiques et leurs sels acceptables du point de vue pharmaceutique.

3. Composition pharmaceutique suivant la revendication 2, **caractérisée en ce que** l'aminométhylchromane est un composé dans lequel
R¹ est l'hydrogène,
R² est l'hydrogène, un reste -OCH₃, -OCH(CH₃)₂, ou -OCH₂C(CH₃)₂-Cl,
ou bien R¹ et R² forment ensemble un reste de formule 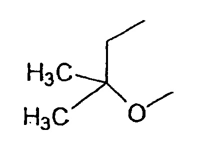
R³ est un reste o-benzosulfimidyle et
n a la valeur 4

4. Composition pharmaceutique suivant la revendication 3, **caractérisée en ce que** l'aminométhylchromane est choisi dans le groupe des composés dans lesquels
R¹ est l'hydrogène et R² est l'hydrogène ou un reste -OCH₃ ;
ou bien
R¹ est l'hydrogène et R² est un reste -OCH(CH₃)₂ ;
ou bien
R¹ est l'hydrogène et R² est un groupe -O-CH₂C(CH₃)₂Cl ;
ou bien
R¹ et R² forment ensemble un reste de formule

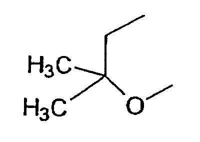

5. Composition pharmaceutique suivant une ou plusieurs des revendications 2 à 4, **caractérisée en ce que** l'aminométhylchromane présente la configuration du (-)-énantiomère.

6. Composition pharmaceutique suivant la revendication 1, **caractérisée en ce que** l'antagoniste des canaux calcium est choisi dans le groupe des antagonistes dihydropyridiniques des canaux calcium.

7. Composition pharmaceutique suivant la revendication 6, **caractérisée en ce que** l'antagoniste des canaux calcium est une dihydropyridine de formule (II) suivante 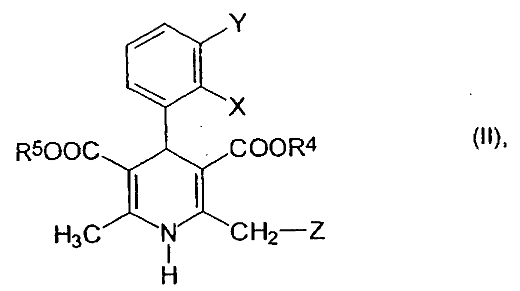 dans laquelle,
X et Y sont identiques ou différents et représentent l'hydrogène, un groupe nitro (-NO₂) ou un halogène, ou bien
X et Y forment, conjointement avec le noyau phényle, un reste benzoxadiazolyle,
R⁴ et R⁵ sont identiques ou différents et représentent un reste alkyle linéaire ou ramifié ayant jusqu'à 6 atomes de carbone, qui peut être substitué par un radical alkoxy ayant jusqu'à 4 atomes de carbone, par un groupe N-benzyl-N-méthylamino ou par un groupe 4-(diphénylméthyl)-pipérazino, ou bien
un groupe de formule 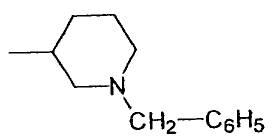 et,
Z représente l'hydrogène ou le groupe -O-CH₂CH₂-NH₂,
ou leurs sels acceptables du point de vue pharmaceutique.

8. Composition pharmaceutique suivant la revendication 7, **caractérisée en ce que** dans la dihydropyridine de formule (II),
X et Y sont identiques ou différents et représentent l'hydrogène, un groupe nitro ou le chlore,
R⁴ et R⁵ sont identiques ou différents et représentent un reste alkyle linéaire ou ramifié ayant jusqu'à 4 atomes de carbone, qui peut être substitué par un radical méthoxy, et
Z représente l'hydrogène.

9. Composition pharmaceutique suivant la revendication 7, **caractérisée en ce que**, dans la dihydropyridine de formule (II),
X est l'hydrogène, Y est un groupe NO₂, Z est l'hydrogène, R⁴ est un reste isopropyle et R⁵ est un reste méthyloxyéthyle.

10. Utilisation de la préparation pharmaceutique suivant l'une quelconque des revendications 1 à 9 pour la fabrication d'un médicament destiné au traitement de l'apoplexie cérébrale (Apoplexia cerebri) et du traumatisme craniocérébral.

11. Nécessaire comprenant, sous une forme séparée, une composition pharmaceutique contenant l'agoniste du récepteur 5-HT_{1A}, choisi dans le groupe des aminométhylchromanes tels que définis dans une ou plusieurs des revendications 1 à 5 et une composition pharmaceutique contenant l'antagoniste des canaux calcium tel que défini dans une ou plusieurs des revendications 6 à 9.
